# EUROPEAN PATENT SPECIFICATION

(11) **EP 1 534 356 B1**
(45) Date of publication and mention of the grant of the patent: **28.07.2010**
(21) Application number: 03785284.5
(22) Date of filing: 13.08.2003
(51) Int. Cl.: A61L 31/02, A61L 31/08, A61L 31/10

(54) **MEDICAL DEVICE EXHIBITING IMPROVED ADHESION BETWEEN POLYMERIC COATING AND SUBSTRATE**
MEDIZINISCHE VORRICHTUNG MIT VERBESSERTER HAFTUNG ZWISCHEN EINEM POLYMEREN BERZUG UND EINEM SUBSTRAT
DISPOSITIF MEDICAL ASSURANT UNE MEILLEURE ADHESION ENTRE UN REVETEMENT POLYMERE ET UN SUBSTRAT

(30) Priority: 13.08.2002 US 403479 P
(43) Date of publication of application: 01.06.2005
(73) Proprietor: Medtronic, Inc., Minneapolis MN 55432-5604 (US)
(72) Inventor: SPARER, Randall, V., Andover, MN 55304 (US); HOBOT, Christopher, M., Tonka Bay, MN 55331 (US); LYU, SuPing, Maple Grove, MN 55311 (US)
(74) Representative: Thomson, James B.
(86) International application number: PCT/US2003/025463
(87) International publication number: WO 2004/014453

(56) References cited:
- WO-A-02/26281
- WO-A-96/23601
- WO-A-98/32474
- ANONYMOUS: "Endoluminal stent with releasable radioisotope" RESEARCH DISCLOSURE, KENNETH MASON PUBLICATIONS, HAMPSHIRE, GB, vol. 447, no. 101, July 2001 (2001-07), XP002265301 ISSN: 0374-4353
- NISHI S ET AL: "Newly developed stent graft with micropored and heparin impregnated SPU film: Long-term follow-up study in vivo" INTERVENTIONAL NEURORADIOLOGY 2001 ITALY, vol. 7, no. SUPPL. 1, 2001, pages 161-166, XP009022986 ISSN: 1123-9344

## Description

### FIELD OF THE INVENTION

The present invention relates to polymer-coated medical devices having improved structural integrity, and related methods.

### BACKGROUND OF THE INVENTION

Polymeric coating of medical devices serves several functions. The surfaces of implantable devices such as catheters or guide wires must be smooth and uniform to assure introduction of such devices without causing trauma to tissue encountered during placement. A polymeric coating may serve as a repository for delivery of an active agent to a subject. For many applications, polymeric coatings must be as thin as possible.

WO 98/32474 discloses coating of a medical device with a thin coherent bond coat which may be coated with additional outer layers. The coatings are baked at elevated temperatures, typically 50°C to 100°C, to drive off the organic solvents used.

The content of WO 03/022323 comprises the state of the art pursuant to Art. 54(3) EPC. This document relates to a stent which includes a polymeric crystalline coating. A primer layer can be formed on the stent which is exposed to heat treatment at a temperature which is less than about the melting temperature (Tₘ) of the selected polymer material.

Prior art coatings suffer from limitations that include structural failure due to cracking and delamination from the device surface. When polymeric coatings are applied from solution, evaporation of the solvent can cause shrinkage with consequent cracking of the coating layer. Water may find its way to the interface between the coating and the device surface, causing further structural damage. This adhesive failure is exacerbated when the coating layers are thin. During the coating process water or solvent molecules may also become trapped at the interface between the coating layer and the substrate, and may be responsible for the formation of cavities, micropores and channels within the coating layer that can lead to premature or uncontrolled release of an active agent from the device. A "skinning" effect is sometimes observed due to the difference in evaporation rates between the solvent near the coating surface and the solvent near the substrate surface. These regions shrink at different rates, causing imperfections in the contact surface between the coating and the substrate and producing stress at the coating/substrate interface, driving delamination.

These problems result at least in part from poor adhesion of the coatings to the substrate surface. Thin polymeric coatings with improved adherence to the surface of medical devices are needed.

### SUMMARY OF THE INVENTION

In one embodiment, the invention provides a method for making a medical device comprising:
applying an undercoat polymer to a substrate surface to form a polymeric undercoat layer;
treating the polymeric undercoat layer to reflow the undercoat polymer and cause formation of a conformable interface between the undercoat layer and the substrate surface; and
applying a top coat polymer to the undercoat layer to form a polymeric top coat layer.

The average thickness of the undercoat layer is preferably less than about 1 micron.

Prior to the application of the top coat layer, the undercoat layer is treated to reflow the undercoat polymer to cause formation of a conformable interface between the undercoat layer and the substrate surface. Reflow of the undercoat polymer is preferably accomplished by heating the undercoat layer to at least about the melt flow temperature of the undercoat polymer for a time sufficient to reflow the polymer. Optionally, the top coat layer comprises an active agent that can be either elutable or non-elutable.

In a preferred embodiment, the top coat layer includes an elutable active agent that elutes from the stent at a slower rate and for a longer duration than the active agent elutes from a comparable stent without the polymeric undercoat layer.

The medical device can be an implantable device, such as a stent, or an extracorporeal device. In a particularly preferred embodiment, the medical device is a stent having a polymeric undercoat layer conformably adherent to a substrate surface of the stent and a polymeric top coat layer adherent to the undercoat layer. The undercoat layer preferably includes a polyurethane, and the top coat layer preferably comprises an active agent.

The invention further includes a medical device prepared by a method as herein described.

The medical device herein described may be used in a method for delivering an active agent to a subject. The method involves contacting the delivery device with a bodily fluid, organ or tissue of a subject to deliver the active agent, wherein the delivery device includes a top coat layer which comprises an active agent. The method can be performed in vivo or ex vivo, depending upon whether the delivery device is an extracorporeal device or an implantable device. The active agent can be elutable or non-elutable. An elutable active agent can elute from the device at a slower rate and for a longer duration than the active agent elutes from a comparable device without the polymeric undercoat layer.

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

### BRIEF DESCRIPTION OF THE DRAWINGS

Figure 1 shows cumulative release of dexamethasone from PVAC/CAB blends that were coated on the surface of SS16L shim without primer treatment. The PVAC/CAB ratio was 100/0 (square), 70/30 (diamond), and 50/50 (triangle).
Figure 2 shows cumulative release of dexamethasone from PVAC/CAB blends that were coated onto the surface of SS16L shim that were treated with PL75D primer that had been subjected to reflow treatment. The PVAC/CAB ratio was 100/0 (square), 70/30 (diamond), and 50/50 (triangle).

### DETAILED DESCRIPTION OF ILLUSTRATIVE EMBODIMENTS

The medical device of the invention is characterized by a substrate surface overlayed with a polymeric layer. This polymeric layer (termed an "undercoat" layer) strongly adheres to the substrate surface and can serve a number of different functions. For example, it can play a role in the electric isolation or thermal isolation of the device, can provide an anti-scratch or abrasion resistant surface and/or can serve as a vehicle for chemical, physical, optical, and/or biological modification of the device surface. This layer is overlayed with another polymeric layer, referred to herein as a top layer or top coat layer. When the device is in use, the outermost layer (i.e., the outermost top coat layer) is in contact with a bodily fluid, organ or tissue of a subject. As a result of a novel fabrication process, the polymeric layers adhere to each other and to the substrate surface in a way that reduces or eliminates cracking and delamination of the polymeric layers. The devices thus exhibit improved structural integrity and safety compared to prior art devices. In devices having top coat layers that contain elutable active agents, the novel construction also yields a reproducible elution profile.

The invention is not limited by the nature of the medical device; rather, any medical device can include the polymeric undercoat layer and top coat layer as described herein. Thus, as used herein, the term "medical device" refers generally to any device that has surfaces that can, in the ordinary course of their use and operation, contact bodily tissue, organs or fluids such as blood. Examples of medical devices include, without limitation, stents, stent grafts, anastomotic connectors leads, needles, guide wires, catheters, sensors, surgical instruments, angioplasty balloons, wound drains, shunts, tubing, urethral inserts, pellets, implants, pumps, vascular grafts, valves, pacemakers, and the like. A medical device can be an extracorporeal device, such as a device used during surgery, which includes, for example, a blood oxygenator, blood pump, blood sensor, or tubing used to carry blood, and the like, which contact blood which is then returned to the subject. A medical device can likewise be an implantable device such as a vascular graft, stent, electrical stimulation lead, heart valve, orthopedic device, catheter, shunt, sensor, replacement device for nucleus pulposus, cochlear or middle ear implant, intraocular lens, and the like. Implantable devices include transcutaneous devices such as drug injection ports and the like.

In general, the materials used to fabricate the medical device of the invention are biomaterials. A "biomaterial" is a material that is intended for implantation in the human body and/or contact with bodily fluids, tissues, organs and the like, and that has the physical properties such as strength, elasticity, permeability and flexibility required to function for the intended purpose. For implantable devices in particular, the materials used are preferably biocompatible materials, i.e., materials that are not overly toxic to cells or tissue and do not cause undue harm to the body.

The invention is not limited by the nature of the substrate surface that is in contact with the polymeric undercoat layer. For example, the substrate surface can be composed of ceramic, glass, metal, polymer, or any combination thereof. In embodiments having a metal substrate surface, the metal is typically iron, nickel, gold, cobalt, copper, chrome, molybdenum, titanium, tantalum, aluminum, silver, platinum, carbon, and alloys thereof. A preferred metal is stainless steel, a nickel titanium alloy, such as NITINOL, or a cobalt chrome alloy, such as NP35N.

Preferably, the substrate surface is not activated or functionalized prior to application of the undercoat layer, although in some embodiments, pretreatment of the substrate surface may be desirable to promote adhesion. Typically, the substrate surface is cleaned with an appropriate solvent to remove surface contamination. The substrate surface can also be cleaned with other methods, such as plasma treatment and thermal treatment.

The polymeric undercoat layer can adhere to the substrate surface by either covalent or non-covalent interactions. Non-covalent interactions include ionic interactions, hydrogen bonding, dipole interactions, hydrophobic interactions and van der Waals interactions, for example. The undercoat layer can be cross-linked or non-cross-linked.

The polymer that is adherent to the substrate surface(generally referred to herein for ease of reference as the undercoat polymer) is preferably a polymer that contains polar groups, however polymers lacking such groups such as styrene or olefin polymers may also be used. Polar groups include hydroxyl, amine, carboxyl, ether, ester, sulfoxide, sulfone, urea, amide, urethane, thiol, carbonate, acetal, carboxylic acid, alkyl halide and combinations thereof. Preferred polymers include polyurethanes, polyesters; polycarbonates, polymethacrylates, polysulfones, polyimides, polyamides, epoxies, polyacetals, vinyl polymers, and blends or copolymers thereof. These polar functional groups facilitate non-covalent bonding with the substrate surface as well as with the optional top coat layer. The undercoat polymer is preferably not a hydrogel.

A particularly preferred undercoat layer consists essentially of a polyurethane. Such a preferred undercoat layer includes a polymer blend that contains polymers other than polyurethane but only in amounts so small that they do not appreciably affect the durometer, durability, adhesive properties, structural integrity and elasticity of the undercoat layer compared to an undercoat layer that is exclusively polyurethane. A particularly preferred undercoat layer includes polyurethane having a Shore durometer hardness of between 50A to 90D, more preferably 55D to 85D, most preferably about 75D. The hardness numbers are derived from the Shore scale, with the A scale being used for softer and the D scale being used for harder materials.

Particularly preferred polymers for use in forming the undercoat layer include polyurethanes available from Thermedics, Inc., Wobum, MA, including polymers marketed under the tradenames TECOPHILIC, TECOPLAST, TECOTHANE, CARBOTHANE, and TECOFLEX. Other preferred polymers include the PELLETHANE and ISOPLAST series available from Dow Chemical Co., Midland MI, especially PELLETHANE 75D; ELASTHANE, PURSIL, CARBOSIL, BIONATE and BIOSPAN, available from the Polymer Technology Group, Inc., Berkeley, CA; ESTANE, available from Noveon, Inc., Cleveland, OH; ELAST-EON, available from AorTech Biomaterials, Frenchs Forest, NSW, Australia; TEXIN, available from Bayer Corporation, Pittsburgh, PA, and other commercially available polymers such as those available from Huntsman Corporation, Salt Lake City, UT.

The invention is not limited by the process used to apply the undercoat polymer to the substrate surface, except that polymerization of the undercoat polymer preferably takes place, in whole or in part, prior to application of the polymer to the substrate surface. Optionally, curing or completion of cross-linking takes place after application of the polymer. Typically, the undercoat polymer is applied to the substrate surface using a solution process, powder coating, melt extrusion, a Langmuir-Blodgett process, gas plasma deposition, chemical vapor deposition or physical vapor deposition. Examples of solution processes include spray coating, dip coating and spin coating. Typical solvents for use in a solution process include tetrahydrofuran (THF), ethanol, methanol, ethylacetate, dimethylformamide (DMF), dimethyacetamide (DMA), dimethylsulfoxide (DMSO), dioxane, N-methyl pyrollidone, chloroform, hexane, heptane, cyclohexane, toluene, formic acid, acetic acid, and/or dichloromethane. Single coats or multiple thin coats of the undercoat polymer can be applied. In a preferred embodiment of the device which includes one or more top coat layers, the undercoat layer is at least partially miscible with the top coat layer.

The coatings or films applied to the substrate surface in accordance with the invention are preferably very thin. The average thickness of the undercoat layer is preferably less than about 2 microns, more preferably less than about 1 micron, even more preferably less than about 0.5 micron, and most preferably less than about 0.1 micron (100 nm). Spin coating can be used to form an undercoat layer having a thickness from 10 nm to 500nm, with an average thickness of less than about 100 nm readily attained; when a Langmuir-Blodgett process is used, the average thickness can be further reduced to less than about 10 nm.

The invention is not limited by the nature of the polymeric top coat layer that is applied to the undercoat layer, or by the process used to apply the top coat polymer to the undercoat layer. The top coat polymer can be selected to render the top coat layer erodable or non-erodable (i.e., biostable), depending on the intended medical application. Single coats or multiple thin coats of the top coat polymer may be applied. Two or more different top coats may be applied. Exemplary polymers and application processes are as described for the undercoat layer, but are not intended to be limited thereby. The top coat polymer can be polymerized, in whole or in part, either before being applied to the device or after being applied to the device. Optionally, curing or completion of cross-linking takes place after application of the top coat layer to the device.

Examples of polymers and polymer blends that can be used to form the undercoat and/or optional top coat layers are described, for example, in U.S. Provisional Patent Application Serial No. 60/403,352, filed on August 13, 2002, and in U.S. Patent Application Serial No. 10/640,853, filed on August 13, 2003.

Optionally, the top coat layer has an active agent incorporated therein (e.g., dispersed or dissolved), preferably a therapeutic agent. If multiple top coat layers are used, the active agent may be incorporated into one or more of those layers. In other embodiments, an active agent may, additionally or alternatively, be incorporated into the undercoat layer.

As used herein, an "active agent" is one that produces a local or systemic effect in a subject (e.g., an animal). Typically, it is a pharmacologically active substance. The term is used to encompass any substance intended for use in the diagnosis, cure, mitigation, treatment, or prevention of disease or in the enhancement of desirable physical or mental development and conditions in a subject. The term "subject" used herein is taken to include humans, sheep, worses, cattle, pigs, dogs, cats, rats, mice, birds, reptiles, fish, insects, arachnids, protists (e.g., protozoa), and prokaryotic bacteria. Preferably, the subject is a human or other mammal.

Active agents can be synthetic or naturally occurring and include, without limitation, organic and inorganic chemical agents, polypeptides (which is used herein to encompass a polymer of L- or D- amino acids of any length including peptides, oligopeptides, proteins, enzymes, hormones, etc.), polynucleotides (which is used herein to encompass a polymer of nucleic acids of any length including oligonucleotides, single-and double-stranded DNA, single- and double-stranded RNA, DNA/RNA chimeras, etc.), saccharides (e.g., mono-, di-, poly-saccharides, and mucopolysaccharides), vitamins, viral agents, and other living material, radionuclides, and the like. Examples include antithrombogenic and anticoagulant agents such as heparin, coumadin, coumarin, protamine, and hirudin; antimicrobial agents such as antibiotics; antineoplastic agents and anti-proliferative agents such as etoposide and podophylotoxin; antiplatelet agents including aspirin and dipyridamole; antimitotics (cytotoxic agents) and antimetabolites such as methotrexate, colchicine, azathioprine, vincristine, vinblastine, fluorouracil, adriamycin, and mutamycinnucleic acids; antidiabetic such as rosiglitazone maleate; and anti-inflammatory agents. Anti-inflammatory agents for use in the present invention include glucocorticoids, their salts, and derivatives thereof, such as cortisol, cortisone, fludrocortisone, Prednisone, Prednisolone, 6α-methylprednisolone, triamcinolone, betamethasone, dexamethasone, beclomethasone, aclomethasone, amcinonide, clebethasol and clocortolone.

In some embodiments, the active agent is elutable from the top coat layer; in other embodiments, the active agent is affixed to or sequestered within the top coat layer. In a preferred embodiment, the active agent is present in a higher concentration in the top coat layer than the undercoat layer.

When a stent or other vascular prosthesis is implanted into a subject, restenosis is often observed during the period beginning shortly after injury to four to six months later; thus for embodiments of the invention that include stents, the generalized elution rates contemplated are such that the active agent, such as a drug, may start to be released immediately after the prosthesis is secured to the lumen wall to lessen cell proliferation. The active agent may continue to elute for days, weeks or months, as desired.

The coating layers are applied to the substrate surface using a novel process that yields a device having improved structural integrity, particularly when exposed to fluids. Prior to application of a top coat layer, the undercoat layer is treated to reflow the undercoat polymer. The device fabrication process thus involves first applying an undercoat polymer to a substrate surface to form the polymeric undercoat layer, followed by treating the polymeric undercoat layer to reflow the undercoat polymer, followed by applying a top coat polymer to the reformed undercoat layer to form the polymeric top coat layer.

Treating the polymeric undercoat layer to reflow the undercoat polymer causes the formation of a "conformable interface" between the undercoat layer and the substrate surface, and also provides a better contact surface for the top coat layer when subsequently applied. The undercoat polymer reflows to fill cavities and imperfections in the substrate surface, thereby effectively increasing the contact area and interactions between the undercoat polymer and the substrate surface. Reflow of the polymer also forces out solvent and/or water molecules that may have been trapped at the interface during application of the undercoat polymer. The interface between the undercoat layer and the substrate surface that results from the reflow process, which exhibits more robust adhesion properties, is referred to herein a "conformable interface." Analogously, after reflow treatment, the polymeric undercoat layer is referred to as "conformably adherent" to the substrate surface. At the interface between the undercoat layer and the substrate surface, the undercoat polymer matches the contours of the substrate surface to exclude water and solvent molecules and maximize interfacial contact.

Reflow of the undercoat polymer can be accomplished in any convenient manner. For example, reflow can be achieved by using thermal treatment, infrared treatment, microwave treatment, RF treatment, mechanical treatment such as compression or shearing, or solvent treatment.

Preferably, the undercoat layer is heated to reflow the undercoat polymer. The undercoat layer is heated to a temperature that is at least as high as the "melt flow temperature" of the undercoat polymer for the time selected to reflow the polymer.

A polymer may exhibit either or both a Tg (the melt temperature for a glass) and a Tm (the melt temperature of a crystal). If a polymer is semi-crystalline, it has both a Tm and a Tg. Tm is greater than Tg. The melt flow temperature for a polymer is above the Tg and/or the Tm of the polymer. If a polymer is amorphous and has no crystallinity, it has only a Tg. The melt flow temperature of amorphous polymers is above the Tg. Tg's can be determined by measuring the mechanical properties, thermal properties, electric properties, etc. as a function of temperature.

It is well-established that physical properties of polymers, such as viscosity, vary with temperature. When the temperature is below the Tg of a polymer, the polymer is said to be in a "glassy state." In a glassy state the polymer is rigid, with a modulus typically within the GPa range. A polymer in a glassy state does not significantly "flow" within a long time frame (years). When the temperature is increased to above the Tg of polymer, the polymer is said to be in a rubbery state. The polymer's modulus in a rubbery state is typically within the MPa range. A polymer in a rubbery state does not significantly flow within a short time frame (hours to days) but may flow on a longer (years) scale. If the temperature is further increased, the polymer chains (i.e., macromolecules) become able to move on a scale of minutes. At this point, the polymer is said to be in a "liquid flow state." The polymer molecules are now able to flow, i.e., deform permanently like a liquid, rather than reversibly deform as in a rubber state. In a liquid flow state, the polymer molecules begin to translocate on the order of minutes, with a consequent movement of their center of mass.

For any time period selected for thermal treatment, the temperature at which the polymer will enter the liquid flow state and reflow during that time period (i.e., the "melt flow temperature") is the preferred minimum temperature that is used to reflow the polymer. It should be clear from the above discussion that melt flow temperature and time are inversely related; the lower the temperature selected for thermal treatment, the longer the time period during which the polymer must be heated in order to cause polymer reflow.

It is typically convenient to reflow the polymer within a period of 5 to 10 minutes (i.e., on the minute scale). To reflow a polyurethane such as PELLETHANE 75D during this time period, a temperature of 215°C to 220°C can be used. On this time scale, reflow of most of the polymers useful as undercoat polymers can be accomplished using a melt flow temperature above about 200°C; for many of them a temperature above about 180°C is sufficient. The melt flow temperature is also a function of molecular weight; i.e., for the same type of polymer and same reflow time, the higher the molecular weight, the higher the melt flow temperature. A skilled artisan can readily determine the melt flow temperature and reflow time for any particular polymer by conducting a few simple melt experiments at different temperatures for the desired time interval.

Typically 1 to 10 minutes is the time period used to reflow the polymer using a thermal treatment in accordance with the invention. It should be cautioned that excessive time at high temperatures is to be avoided as the polymer can begin to degrade.

Surprisingly, when an elutable active agent is included in the top coat layer, the medical device of the invention exhibits improved release characteristics. In particular, the active agent elutes from the device at a slower rate and for a longer duration than it elutes from a comparable device lacking the polymeric undercoat layer. In addition, the elution profile is more reproducible. Without being bound by theory, it is believed that the kinetics of elution are affected by the primer coat. Cracking and delamination are reduced, eliminating routes for premature release of the active agent. It is believed that the agent elutes more uniformly from the surface of the top coat in contact with the body fluid, organ or tissue (i.e., the interface between the device and the tissue, organ or fluid) as intended, rather than from the undercoat/substrate interface due to delamination or through cracks in the coating surface.

### EXAMPLES

The present invention is illustrated by the following examples. It is to be understood that the particular examples, materials, amounts, and procedures are to be interpreted broadly in accordance with the scope and spirit of the invention as set forth herein.

### Example I (comparative).

### Untreated poly(etherurethane) coating applied to a metal surface

Poly(etherurethane) (PELLETHANE) 75D (Dow Chemical Co., Midland, MI) was cast from 1 wt% tetrahydrofuran (THF) solution to a bare stainless steel (316L) shim surface. Specifically, PELLETHANE 75D was dried overnight at 70°C under reducer pressure, then melted and pressed between two hot plates at 230°C for 5-10 minutes. After the films were cooled in air, a tetrahydrofuran (THF) solution with 1 wt-% of PELLETHANE 75D was made by dissolving the films in anhydrous THF at about 25°C by stirring with a magnetic bar overnight. The solution was coated onto a bare stainless steel (316L) shim surface that was cleaned by rinsing with THF. The coating was dried in a moisture free environment by purging with N₂ gas.

The coated shim was immersed in phosphate buffered saline solution (PBS, potassium phosphate monobasic (NF tested), 0.144 g/L, sodium chloride (USP tested), 9 g/L, and sodium phosphate dibasic (USP tested) 0.795 g/L, pH = 7.0 to 7.2 at 37°C, purchased from HyClone, Logan, UT) and shaken periodically by hand. After about 5 minutes, the coating delaminated from the shim. This demonstrated that without primer pretreatment, the adhesion between the polymer coating and stainless steel was poor in PBS.

### Example II.

### Thermal treatment of poly(etherurethane) coating applied to a metal surface

The PELLETHANE-coated 316L shim made in Example I was thermally pretreated by heating it at 215°C to 220 °C for 5 to 10 minutes in air or, preferably, inert gas (i.e., N₂). Then the treated shim was cooled to room temperature and was immersed in PBS for more than 1 month. The coating strongly adhered to the metal surface and did not delaminate during that time period. This thermally treated PELLETHANE 75D coating is referred to in the following examples as "PELLETHANE primer," and surfaces that have been coated with a primer (whether PELLETHANE or another primer) then thermally treated as in this example are referred to as "primed surfaces." Primed surfaces that have not been thermally treated (such as in Example IV) will be specifically indicated.

### Example III (comparative).

### Poly(caprolactone) (PCL) coating applied to a metal surface

PCL was coated from a 1 wt% THF solution to a bare stainless steel (316L) shim surface. The coating was dried in a moisture free environment by purging with N₂ gas. The coated shim was immersed in PBS solution and periodically shaken by hand. The PCL film delaminated from substrate within about 2 minutes. The adhesion between the 316L shim and PCL was poor.

### Example IV (comparative).

### PCL coating applied to a metal surface primed but not thermally treated

PELLETHANE (PL75D) was coated onto the surface of a 316L shim without thermal treatment. PCL was then coated from 1wt% of THF solution onto the primed metal surface. The coating was dried in a moisture free environment by purging with N₂ gas. The coated shim was immersed in PBS solution at room temperature and periodically shaken by hand. The PCL film delaminated from substrate within about 2 minutes. The non-thermally treated PL75D layer did not promote the adhesion between PCL and the 316L shim.

### Example V.

### PCL coating applied to a primed metal surface (PELLETHANE)

PELLETHANE (PL75D) was coated onto the surface of a 316L shim and thermally treated as in Example II (e.g., 215 - 220 °C for 5 to 10 minutes). PCL was then coated from 1wt% of THF solution onto the primed metal surface. The coating was dried in a moisture free environment by purging with N₂ gas. The coated shim was immersed in PBS solution and periodically shaken by hand for about 5 minutes. The PCL film did not delaminate from substrate. Then the sample was left in PBS at room temperature for 12 days. No delamination was observed. The thermally treated primer thus promoted the adhesion between PCL and the 316L shim.

### Example VI.

### Drug-loaded poly(vinyl acetate) (PVAC)/ cellulose acetate butyrate (CAB) blend applied to a primed metal surface (PELLETHANE primer)

Various PVAC/CAB blends loaded with about 10 wt% of dexamethasone were coated onto PELLETHANE primer treated (Example II) and nontreated 316L shims and the samples were dried. The coatings having a CAB fraction more than 30 wt% delaminated from the nontreated shims within the first two hours of immersion in PBS at 37 °C. However, no delamination was observed from the treated surfaces even after 1 month of immersion in PBS at the same temperature.

### Example VII.

### PVAC/CAB blends applied to primed stent surface (PELLETHANE primer)

A PVAC/CAB (50/50) blend was coated onto PELLETHANE primer treated (as in Example II) and nontreated 316L stents (S7, Medtronic AVE, Santa Rosa, CA), and the samples were dried. A scratch test showed that the coating in the primer treated cases adhered to the treated stents much more strongly than the un-treated stents. The average thickness of the primers on the stents was about 0.4 to 1.0 microns.

### Example VIII.

### Drug (Dexamethasone)-loaded poly(carbonate urethane)/polycarbonate (PCU/PC) blends applied to a primed metal surface (PELLETHANE primer)

Various PCU/PC blends loaded with about 10 wt% of dexamethasone were coated onto PELLETHANE primer treated (Example II) and nontreated 316L shims. Most of the coatings delaminated from the untreated shims within the first 2 days of immersion in PBS and all of them delaminated after 6 more days. However, there was essentially no delamination from the treated shims under the same conditions after at least 4 weeks.

### Example IX.

### PCU/PC blends applied to a primed metal stent surface (PELLETHANE primer)

PCU/PC blends (100/0, 95/5, and 50/50) were coated onto 316L stents with and without PELLETHANE primer (0.7 - 1.2 micron thick). A scratch test showed that the coatings in the primer-treated stents adhered more strongly than that in the untreated stents.

### Example X.

### Drug-loaded PELLETHANE 75D (PL75D)/TECOPLAST blend applied to a primed metal stent surface (PELLETHANE primer)

PL75D/TECOPLAST blends loaded with a low molecular weight, hydrophobic active agent were coated onto PELLETHANE primer treated stents (Example II) from a THF solution. The coatings were durable and no delamination was observed during immersion in PBS at 37 °C for at least 14 days.

### Example XI.

### Polycarbonate (PC) coating applied to a primed metal surface (poly(carbonate urethane) primer)

An 316L metal shim was pretreated with poly(carbonate urethane) primer coating using the procedure described in Example II for PELLETHANE 75D primer. Polycarbonate was coated onto the primed surface, and also onto shims that were not pretreated. The coating adhered to the pretreated shim very well and no delamination was observed during immersion in PBS at room temperature for at least 4 weeks. If the shims were not pretreated with a PCU primer (or other primer), the PC film delaminated within 5 - 10 minutes of immersion in PBS.

### Example XII.

### Drug-loaded TECOPHILIC/poly(vinyl acetate-co-vinyl pyrrolidone) (PVP-VA) coating applied to a primed metal stent surface (TECOPHILIC primer)

A 316L stent was pretreated with TECOPHILIC (polyethylene oxide urethane) (TCPL) primer using the same procedure as that for the PELLETHANE primer in Example II. PVP-VA was from Sigma-Aldrich Chemical Company, Milwaukee, WI. TCPUPVP-VA loaded with RESTEN NG (7,000 g/mol molecular weight and water-soluble antisense oligonuctleotide. AVI Biopoharma, Corvallis, Oregon) was coated onto the primed surface. The stents were subjected to a durability test during which they were mounted on a balloon catheter (Medtronic AVE, Santa Rosa, CA) and passed down a PBS-filled 2 mm (i.d.) J-bended catheter (Medtronic AVE, Santa Rosa, CA), then radially expanded in PBS. The stents were viewed using optical microscopy and, in some instances, scanning electron microscopy (SEM). No delamination was observed.

### Example XIII.

### Polycarbonate coating applied to a primed metal surface (TECOPLAST primer)

A 316L shim was pretreated with TECOPLAST (polyether urethane) (TCPT) primer using the same procedure as that for the PELLETHANE primer in Example II. Polycarbonate (PC) was coated onto the primed surface. The PC film adhered to the shim very well and no delamination was observed after the coating immersed in PBS for at least 1 month.

### Example XIV.

### Improved drug release properties from poly(vinyl acetate) (PVAC)/cellulose acetate butyrate (CAB) blend coating applied to a primed metal surface (PELLETHANE primer)

Mixtures of PVAC and CAB with weight ratios varying from 100/0 to 50/50, each loaded with about 10 wt% of dexamethasone. The solutions were cast the solutions onto the surface of bare SS16L shims (control) and PL75D primer treated SS16L shims (as in Example II). After the samples completely dried under nitrogen gas, dexamethasone dissolution tests were conducted in PBS solution (3 mL) at 37° C. During testing, the vials were shaken at a rate of about 10 times per minute. The dissolution solution for each sample was refreshed at various times. The eluted dexamethasone was measured with a UV-Vis spectroscopy (HP 4152A). The cumulative drug release per area of sample size was plotted as a function of square root of time. Theoretically, cumulative release per area should be a linear function of square root of time at the early stage of release. Then the release rate decreases exponentially, approaching zero at infinitely long time.

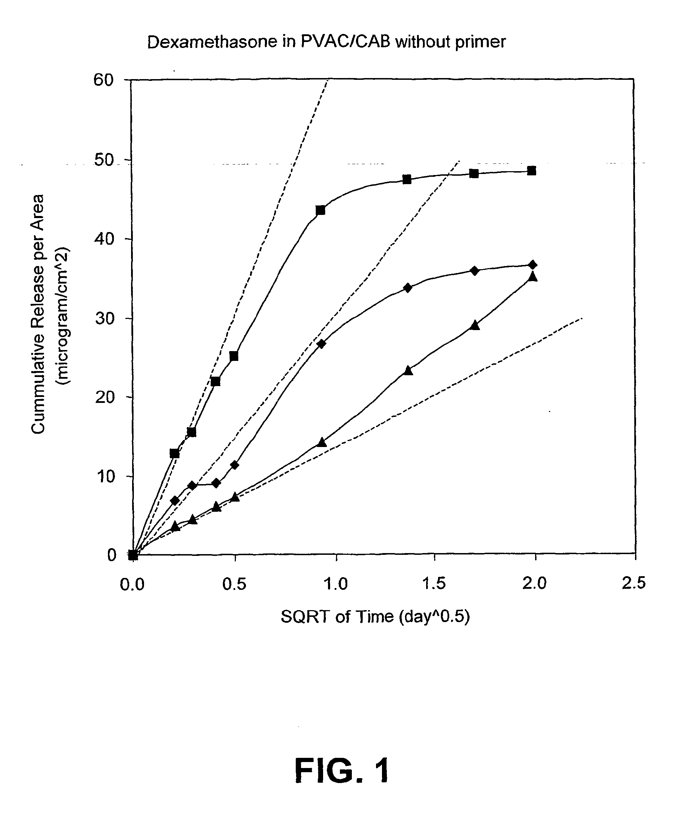

For the samples on the bare shims, the release curves are plotted in Figure 1. For each blend (PVAC/CAB 100/0, 70/30 and 50/50), the elution profile observed during the initial elution period differed from the expected linear trend (shown as dashed lines in Fig. 1). It is believed that this deviation is attributable to delamination of the coating from the shim. Delamination causes the drug to release from the device prematurely, and therefore increases the rate of drug delivery.

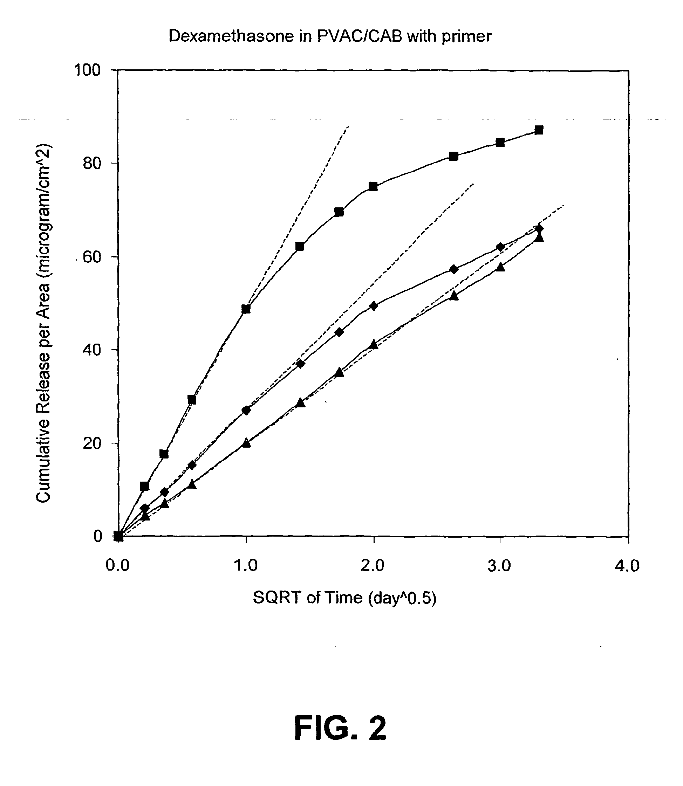

Release curves for sample on the primer treated shim are shown in Figure 2. The observed cumulative release for each different PVAC/CAB blend was a linear function of the square root of time at early stage then slowed down, as would be expected from theory. Therefore, the primer improved the release properties of drug from the thin polymer films.

## Claims

1. A method for making a medical device comprising:
applying an undercoat polymer to a substrate surface to form a polymeric undercoat layer;
treating the polymeric undercoat layer to reflow the undercoat polymer and cause formation of a conformable interface between the undercoat layer and the substrate surface; and
applying a top coat polymer to the undercoat layer to form a polymeric top coat layer.

2. The method of claim 1 wherein the undercoat polymer comprises polar groups selected from the group consisting of hydroxyl, amine, carboxyl, ether, ester, sulfoxide, sulfone, urea, amide, urethane, thiol, carbonate, acetal, carboxylic acid, alkyl halide and combinations thereof.

3. The method of claim 1 or claim 2 wherein the average thickness of the undercoat layer is less than about 1 micron.

4. The method of any one of claims 1 to 3 wherein the undercoat polymer is applied to the substrate surface using a technique selected from the group consisting of a solution process, powder coating, melt extrusion, vapor deposition, and a Langmuir-Blodgett process.

5. The method of claim 4 wherein the solution process comprises spray coating, dip coating, or spin coating.

6. The method of any preceding claim wherein the polymeric undercoat layer comprises at least one polymer selected from the group consisting of a polyurethane, a polyester, a polycarbonate, a polymethacrylate, a polysulfone, a polyimide, a polyamide, a linear epoxy, a polyacetal, a vinyl polymer, and any blend or copolymer thereof.

7. The method of any one of claims 1 to 5 wherein the polymeric undercoat layer comprises a polyurethane.

8. The method of any preceding claim wherein the undercoat layer does not comprise a hydrogel.

9. The method of any preceding claim wherein the undercoat layer is not cross-linked.

10. The method of any preceding claim wherein the undercoat polymer is polymerized prior to application to the substrate surface.

11. The method of any preceding claim wherein the undercoat layer is treated to reflow the undercoat polymer using a technique selected from the group consisting of thermal treatment, infrared treatment, microwave treatment, RF treatment, mechanical treatment and solvent treatment.

12. The method of any one of claims 1 to 10 wherein the undercoat layer is heated to at least about the melt flow temperature of the undercoat polymer for a time sufficient to reflow the polymer.

13. The method of any one of claims 1 to 10 wherein treating the undercoat layer to reflow the undercoat polymer comprises heating the undercoat layer to at least about 200°C for a time period sufficient to reflow the undercoat polymer.

14. The method of any preceding claim wherein the top coat layer comprises an active agent.

15. The method of claim 14 wherein the active agent is present in a higher concentration in the top coat layer than the undercoat layer.

16. The method of claim 14 or claim 15 wherein the active agent is selected from the group consisting of an anti-thrombogenic agent, an anticoagulant agent, an antimicrobial agent, an anti-neoplastic agent, an anti-proliferative agent, an antiplatelet agent, an antimetabolite, and an anti-inflammatory agent.

17. The method of any preceding claim wherein the substrate surface comprises a material selected from the group consisting of ceramic, glass, metal and a polymer.

18. The method of claim 17 wherein the metal is selected from the group consisting of iron, nickel, gold, cobalt, copper, chrome, molybdenum, titanium, tantalum, aluminum, silver, platinum, carbon, and alloys thereof.

19. The method of claim 18 wherein the alloy is stainless steel, a nickel titanium alloy, or a cobalt chrome alloy.

20. The method of any preceding claim wherein the substrate surface is not activated or functionalized prior to application of the undercoat layer.

21. A medical device prepared by a method as claimed in any one of claims 1 to 20.

22. The medical device of claim 21 which is an implantable device.

23. The medical device of claim 21 which is an extracorporeal device.

24. The medical device of claim 21 selected from the group consisting of a stent, stent graft, anastomatic connector, lead, needle, guide wire, catheter, sensor, surgical instrument, angioplasty balloon, wound drain, shunt, tubing, urethral insert, pellet, implant, blood oxygenator, pump, vascular graft, valve, pacemaker, orthopedic device, replacement device for nucleus pulposus, and intraocular lense.

25. The medical device of claim 21 which is a stent.

## Patentansprüche

1. Ein Verfahren zur Herstellung einer medizinischen Vorrichtung, das folgendes umfasst:
Aufbringen eines Unterschichtpolymers auf eine Substratoberfläche zur Bildung einer polymeren Unterschicht;
Behandeln der polymeren Unterschicht zur Wiederaufschmelzung des Unterschichtpolymers und Herbeiführung der Bildung einer konformen Schnittstelle zwischen der Unterschicht und der Substratoberfläche; und
Aufbringen eines Deckschichtpolymers auf die Unterschicht zur Bildung einer polymeren Deckschicht.

2. Das Verfahren nach Anspruch 1, wobei das Unterschichtpolymer polare Gruppen umfasst, die aus der Gruppe ausgewählt sind, die aus Hydroxyl, Amin, Carboxyl, Ether, Ester, Sulfoxid, Sulfon, Carbamid, Amid, Urethan, Thiol, Carbonat, Acetal, Carbonsäure, Alkylhalogenid und Kombinationen davon besteht.

3. Das Verfahren nach Anspruch 1 oder Anspruch 2, wobei die durchschnittliche Dicke der Unterschicht weniger als etwa 1 µm beträgt.

4. Das Verfahren nach einem der Ansprüche 1 bis 3, wobei das Unterschichtpolymer auf die Substratoberfläche unter Verwendung eines Verfahrens aufgebracht wird, das aus der Gruppe ausgewählt ist, die aus einem Löseprozess, einer Pulverbeschichtung, einer Schmelzextrusion, einer Gasphasenabscheidung und einem Langmuir-Blodgett-Prozess besteht.

5. Das Verfahren nach Anspruch 4, wobei der Löseprozess Sprühbeschichtung, Tauchbeschichtung oder Schleuderbeschichtung umfasst.

6. Das Verfahren nach einem vorstehenden Anspruch, wobei die polymere Unterschicht mindestens ein Polymer umfasst, das aus der Gruppe ausgewählt ist, die aus einem Polyurethan, einem Polyester, einem Polycarbonat, einem Polymethacrylat, einem Polysulfon, einem Polyimid, einem Polyamid, einem linearen Epoxid, einem Polyacetal, einem Vinylpolymer und einem beliebigen Blend oder Copolymer davon besteht.

7. Das Verfahren nach einem der Ansprüche 1 bis 5, wobei die polymere Unterschicht ein Polyurethan umfasst.

8. Das Verfahren nach einem vorstehenden Anspruch, wobei die Unterschicht kein Hydrogel umfasst.

9. Das Verfahren nach einem vorstehenden Anspruch, wobei die Unterschicht nicht vernetzt ist.

10. Das Verfahren nach einem vorstehenden Anspruch, wobei das Unterschichtpolymer vor der Aufbringung auf die Substratoberfläche polymerisiert ist.

11. Das Verfahren nach einem vorstehenden Anspruch, wobei die Unterschicht zur Wiederaufschmelzung des Unterschichtpolymers unter Verwendung eines Verfahrens behandelt wird, das aus der Gruppe ausgewählt ist, die aus Wärmebehandlung, Infrarotbehandlung Mikrowellenbehandlung, HF-Behandlung, mechanischer Behandlung und Lösungsmittelbehandlung besteht.

12. Das Verfahren nach einem der Ansprüche 1 bis 10, wobei die Unterschicht auf mindestens etwa Schmelztemperatur des Unterschichtpolymers für eine zur Wiederaufschmelzung des Polymers ausreichende Zeit erwärmt wird.

13. Das Verfahren nach einem der Ansprüche 1 bis 10, wobei die Behandlung der Unterschicht zur Wiederaufschmelzung des Unterschichtpolymers das Erwärmen der Unterschicht auf mindestens etwa 200 °C für eine zur Wiederaufschmelzung des Unterschichtpolymers ausreichende Zeitdauer umfasst.

14. Das Verfahren nach einem vorstehenden Anspruch, wobei die Deckschicht einen Wirkstoff umfasst.

15. Das Verfahren nach Anspruch 14, wobei der Wirkstoff in einer höheren Konzentration in der Deckschicht als in der Unterschicht vorhanden ist.

16. Das Verfahren nach Anspruch 14 oder Anspruch 15, wobei der Wirkstoff aus der Gruppe ausgewählt ist, die aus einem anti-thrombogenen Mittel, einem gerinnungshemmenden Mittel, einem anti-mikrobiellen Mittel, einem anti-neoplastischen Mittel, einem anti-proliferativen Mittel, einem Antiplättchenmittel, einem Antimetaboliten und einem entzündungshemmenden Mittel besteht.

17. Das Verfahren nach einem vorstehenden Anspruch, wobei die Substratoberfläche ein Material umfasst, das aus der Gruppe ausgewählt ist, die aus Keramik, Glas, Metall und einem Polymer besteht.

18. Das Verfahren nach Anspruch 17, wobei das Metall aus der Gruppe ausgewählt ist, die aus Eisen, Nickel, Gold, Cobalt, Kupfer, Chrom, Molybdän, Titan, Tantal, Aluminium, Silber, Platin, Kohlenstoff und Legierungen davon besteht.

19. Das Verfahren nach Anspruch 18, wobei die Legierung Edelstahl, eine Nickel-Tantal-Legierung oder eine Cobalt-Chrom-Legierung ist.

20. Das Verfahren nach einem vorstehenden Anspruch, wobei die Substratoberfläche vor der Aufbringung auf die Unterschicht nicht aktiviert oder funktionalisiert ist.

21. Eine medizinische Vorrichtung, die nach einem Verfahren wie in einem der Ansprüche 1 bis 20 beansprucht hergestellt ist.

22. Die medizinische Vorrichtung nach Anspruch 21, die eine implantierbare Vorrichtung ist.

23. Die medizinische Vorrichtung nach Anspruch 21, die eine extrakorporale Vorrichtung ist.

24. Die medizinische Vorrichtung nach Anspruch 21, die aus der Gruppe ausgewählt ist, die aus einem Stent, einem Stenttransplantat, einer anastomotischen Verbindung, einer Führung, einer Nadel, einem Führungsdraht, einem Katheter, einem Sensor, einem chirurgischen Instrument, einem Angioplastie-Ballon, einem Abflussschlauch für Wunden, einem Shunt, einer Rohrleitung, einem Harnröhreneinsatz, einem Pellet, einem Implantat, einem Blutoxygenator, einer Pumpe, einem vaskulären Transplantat, einem Ventil, einem Pacemaker, einer orthopädischen Vorrichtung, einer Austauschvorrichtung für den Nucleus pulposus und einer intraokularen Linse besteht.

25. Die medizinische Vorrichtung nach Anspruch 21, die ein Stent ist.

## Revendications

1. Procédé de fabrication d'un dispositif médical comprenant :
- l'application d'un polymère de sous-couche à une surface de substrat pour former une sous-couche polymère :
- le traitement de la sous-couche polymère pour provoquer une refusion du polymère de sous-couche et provoquer une formation d'une interface concordante entre la sous-couche et la surface de substrat ; et
- l'application d'un polymère de couche de finition à la sous-couche pour former une couche de finition polymère.

2. Procédé selon la revendication 1, dans lequel le polymère de sous-couche comprend des groupes polaires choisis dans le groupe constitué par hydroxyle, amine, carboxyle, éther, ester, sulfoxyde, sulfone, urée, amide, uréthane, thiol, carbonate, acétal, acide carboxylique, halogénure d'alkyle et leurs combinaisons.

3. Procédé selon la revendication 1 ou la revendication 2, dans lequel l'épaisseur moyenne de la sous-couche est inférieure à environ 1 micron.

4. Procédé selon l'une quelconque des revendications 1 à 3, dans lequel le polymère de sous-couche est appliqué à la surface de substrat à l'aide d'une technique choisie dans le groupe constitué par un procédé en solution, un revêtement pulvérulent, une extrusion de matière fondue, un dépôt en phase vapeur et un procédé de Langmuir-Blodgett.

5. Procédé selon la revendication 4, dans lequel le procédé en solution comprend un revêtement par pulvérisation, un revêtement par immersion ou un dépôt à la tournette.

6. Procédé selon l'une quelconque des revendications précédentes, dans lequel la sous-couche polymère comprend au moins un polymère choisi dans le groupe constitué par un polyuréthane, un polyester, un polycarbonate, un polyméthacrylate, une polysulfone, un polyimide, un polyamide, un époxy linéaire, un polyacétal, un polymère vinylique et tout mélange ou copolymère de ceux-ci.

7. Procédé selon l'une quelconque des revendications 1 à 5, dans lequel la sous-couche polymère comprend un polyuréthane.

8. Procédé selon l'une quelconque des revendications précédentes, dans lequel la sous-couche ne comprend pas d'hydrogel.

9. Procédé selon l'une quelconque des revendications précédentes, dans lequel la sous-couche n'est pas réticulée.

10. Procédé selon l'une quelconque des revendications précédentes, dans lequel le polymère de sous-couche est polymérisé avant application à la surface de substrat.

11. Procédé selon l'une quelconque des revendications précédentes, dans lequel la sous-couche est traitée pour provoquer une refusion du polymère de sous-couche à l'aide d'une technique choisie dans le groupe constitué par un traitement thermique, un traitement infrarouge, un traitement aux micro-ondes, un traitement radiofréquence, un traitement mécanique et un traitement par solvant.

12. Procédé selon l'une quelconque des revendications 1 à 10, dans lequel la sous-couche est chauffée à au moins environ la température de fusion du polymère de sous-couche pendant un temps suffisant pour provoquer une refusion du polymère.

13. Procédé selon l'une quelconque des revendications 1 à 10, dans lequel le traitement de la sous-couche pour provoquer une refusion du polymère de sous-couche comprend le chauffage de la sous-couche à au moins environ 200°C pendant une période de temps suffisante pour provoquer une refusion du polymère de sous-couche.

14. Procédé selon l'une quelconque des revendications précédentes, dans lequel la couche de finition comprend un agent actif.

15. Procédé selon la revendication 14, dans lequel l'agent actif est présent dans une concentration plus grande dans la couche de finition que dans la sous-couche.

16. Procédé selon la revendication 14 ou la revendication 15, dans lequel l'agent actif est choisi dans le groupe constitué par un agent anti-thrombogène, un agent anticoagulant, un agent antimicrobien, un agent anti-néoplasique, un agent anti-prolifératif, un agent anti-agrégant plaquettaire, un antimétabolite et un agent anti-inflammatoire.

17. Procédé selon l'une quelconque des revendications précédentes, dans lequel la surface de substrat comprend un matériau choisi dans le groupe constitué par la céramique, le verre, le métal et un polymère.

18. Procédé selon la revendication 17, dans lequel le métal est choisi dans le groupe constitué par le fer, le nickel, l'or, le cobalt, le cuivre, le chrome, le molybdène, le titane, le tantale, l'aluminium, l'argent, le platine, le carbone et leurs alliages.

19. Procédé selon la revendication 18, dans lequel l'alliage est l'acier inoxydable, un alliage nickel-titane ou un alliage cobalt-chrome.

20. Procédé selon l'une quelconque des revendications précédentes, dans lequel la surface de substrat n'est pas activée ou fonctionnalisée avant application de la sous-couche.

21. Dispositif médical préparé par un procédé tel que défini à l'une quelconque des revendications 1 à 20.

22. Dispositif médical selon la revendication 21, qui est un dispositif implantable.

23. Dispositif médical selon la revendication 21, qui est un dispositif extracorporel.

24. Dispositif médical selon la revendication 21, choisi dans le groupe constitué par un stent, une greffe de stent, un connecteur anastomatique, une dérivation, une aiguille, un fil-guide, un cathéter, un capteur, un instrument chirurgical, un ballonnet d'angioplastie, un drain de plaie, un shunt, une tubulure, un insert urétral, une pastille, un implant, un oxygénateur de sang, une pompe, une greffe vasculaire, une valvule, un stimulateur cardiaque, un dispositif orthopédique, un dispositif de remplacement pour noyau gélatineux et une lentille intraoculaire.

25. Dispositif médical selon la revendication 21, qui est un stent.
